# EUROPÄISCHE PATENTANMELDUNG

(11) **EP 2 292 588 A1**
(43) Veröffentlichungstag der Anmeldung: **09.03.2011**
(21) Anmeldenummer: 09008828.7
(22) Anmeldetag: 06.07.2009
(51) Int. Cl.: C07C 235/08, A61K 31/164, A61P 17/00, A61K 8/68, A61Q 19/00, C07J 9/00, C07J 31/00, C07J 41/00, C07J 51/00

(54) **Hybridmoleküle aus Lipiden und Lipidanalogen Verbindungen und deren Verwendung als Arzneimittel oder kosmetische Zubereitung**

(30) Priorität: 23.06.2009 US 219558 P; 23.06.2009 EP 09008218
(71) Anmelder: Wolf, Hans Uwe, 89231 Neu-Ulm (DE)
(72) Erfinder: Wolf, Hans Uwe, 89231 Neu-Ulm (DE)
(74) Vertreter: Pfenning, Meinig & Partner GbR

(57) **Zusammenfassung**

Die Erfindung betrifft Hybridmoleküle aus zwei Lipiden oder Lipid-analogen Verbindungen, die über ihr lipophiles Ende miteinander verknüpft sind. Die Hybridmoleküle weisen dabei an ihrem hydrophilen Ende mindestens eine hydrophile Gruppe zur Vergrößerung der Hydrathülle des Hybridmoleküls auf. Die erfindungsgemäßen Hybridmoleküle können als Arzneimittel oder als kosmetische Zubereitung eingesetzt werden.

## Beschreibung

Die Erfindung betrifft Hybridmoleküle aus zwei Lipiden oder Lipid-analogen Verbindungen, die über ihr lipophiles Ende miteinander verknüpft sind. Die Hybridmoleküle weisen dabei an ihrem hydrophilen Ende mindestens eine hydrophile Gruppe zur Vergrößerung der Hydrathülle des Hybridmoleküls auf. Die erfindungsgemäßen Hybridmoleküle können als Arzneimittel oder als kosmetische Zubereitung eingesetzt werden.

Grundsätzlich enthalten alle biologischen Membranen, insbesondere Zellmembranen, als essentielle Bestandteile sog. Lipide und Lipid-analoge Substanzen, die strukturell unterschiedlich aufgebaut sind, die sich jedoch in ihrer prinzipiellen Bauweise ähneln. Die prinzipielle Ähnlichkeit der Struktur besteht darin, dass sie aus einem lipophilen (hydrophoben) und einem hydrophilen (lipophoben) Molekülteil aufgebaut sind, dass sie somit eine sog. amphiphile Struktur besitzen, die zum Teil sehr stark ausgeprägt ist.

Die amphiphile Struktur der Lipide und Lipid-analogen Substanzen, d.h. die gleichzeitige Anwesenheit eines (stark) hydrophoben und eines hydrophilen, polaren Anteils der Molekülstruktur, führt dazu, dass sich die Lipide und Lipid-analogen Substanzen in einer wässrigen Phase (meist zusammen mit anderen Lipiden) spontan zu einer Lipid-Doppelschicht, einem so genannten "Lipid-Bilayer" anordnen, der u. a. die Grundlage der Struktur biologischer Membranen darstellt. Das Bauprinzip dieser Doppelschicht ist für alle Lipide und Lipid-analogen Substanzen gleich: Sie ordnen sich in zwei parallelen, eng zusammenliegenden Schichten an, wobei sich jeweils die hydrophoben Reste der betreffenden Moleküle im Innern der Membran direkt gegenüberliegen und in Kontakt treten. Sie bilden somit den hydrophoben inneren Bereich der Membrandoppelschicht, während die hydrophilen Reste auf beiden Seiten der Lipid-Doppelschicht mit der wässrigen Phase des Extra- und des Intrazellularraumes in Kontakt stehen. Die Tendenz zur Ausbildung dieser Lipid-Doppelschicht besteht sowohl innerhalb als auch außerhalb eines Organismus, z. B. in einem wässrigen System, in welchem die Eigenschaften der Lipid-Doppelschichten in eigens hierzu ausgelegten experimentellen Anordnungen untersucht werden können.

Die Gruppe der Lipide und Lipid-analogen Substanzen setzt sich im Wesentlichen aus verschiedenen Ceramiden mit unterschiedlicher Struktur (Ceramid 1 - 9), freien Fettsäuren (insb. Palmitinsäure), Cholesterol und verschiedenen Cholesterolestern, wie z.B. Cholesterolsulfat zusammen.

Im Fall der Ceramide besteht der lipophile Bereich aus dem Alkanrest der Sphingosin-Grundstruktur und dem an der NH-Gruppe des Sphingosins angekoppelten Fettsäurerest (Acylrest), während der hydrophile Bereich durch die beiden OH-Gruppen und durch die -NH-CO- Struktur des Sphingosin-Grundkörpers gebildet wird.

Im Fall der Phospholipide besteht der lipophile Bereich aus den beiden Alkanresten der in 2- und 3-Position des Glycerins über eine Esterbindung gebundenen Fettsäuren, während der hydrophile Bereich durch das Glycerin und die in 1-Position des Glycerins gebundene Phosphatgruppe sowie die an der Phosphatgruppe gebundenen weiteren Komponenten Serin, Ethanolamin, Cholin und Inositol gebildet wird.

Im Fall der Glykolipide (Cerebroside) besteht der lipophile Bereich aus dem Alkanrest der Sphingosin-Grundstruktur und dem Alkan- oder Alken-Rest der an der NH₂-Gruppe gebundenen Fettsäure, während der hydrophile Bereich durch die beiden OH-Gruppen und durch die -NH-CO-Struktur des Sphingosin-Grundkörpers sowie durch die an der Hydroxymethylengruppe gebundene Monosaccharid-Einheit gebildet wird.

Im Fall der geradkettigen, unverzweigten gesättigten und ungesättigten Fettsäuren wie z.B. der Palmitinsäure und der Linolsäure besteht der hydrophobe Molekülbereich aus dem Alkan- oder Alken-Rest der Fettsäure, während der hydrophile Anteil eine Carboxylgruppe ist.

Im Fall des Cholesterols und seiner Ester sowie des Lanosterols und des Sitosterols besteht der lipophile Teil der Moleküle aus dem Cyclopentano-perhydrophenanthren-Grundgerüst mit der in Position 17 gebundenen verzweigten aliphatischen Seitenkette, während die hydrophile Struktur die in Position 3 des Ringsystems stehende OH-Gruppe ist, die überdies noch mit dem stark hydrophilen Sulfat verestert sein kann.

Die Struktur der Lipid-Doppelschicht in einem Organismus bildet sich spontan aus. Obwohl sie eine erhebliche Stabilität besitzt, besteht z. B. bei Vorliegen einer Lipid-Stoffwechselstörung die Möglichkeit, dass eine biologische Membran einen Teil ihrer Lipid-Komponenten verliert, weil diese Moleküle entweder zu langsam und/oder in einem unzureichenden Ausmaß gebildet oder zu schnell verstoffwechselt werden. Dadurch verarmen die betreffenden Membranen an den jeweiligen Komponenten, was u. a. zu einer Störung der Membranstruktur und -funktion führt.

Die physiologische Zusammensetzung der Membran-Lipide des Stratum corneum der menschlichen Haut ist allerdings noch aus einem zweiten Grund für die normale Struktur und Funktion der Haut von essentieller Bedeutung. Die Anwesenheit eines ausreichenden Gehalts an diesen Lipiden gewährleistet die uneingeschränkte Fähigkeit der Haut zur Bindung einer physiologischen Menge an Wasser. Der Verlust eines Teils der Stratum-corneum-Lipide führt daher zu einer Einschränkung der Wasserbindefähigkeit, was durch die sog. Corneometrie klinisch feststellbar ist. Weiterhin erhöht sich im Zuge des Lipidverlustes der sog. Transepidermale Wasserverlust (Trans Epidermal Water Loss = TEWL) der Haut. Dies zeigt sich im Auftreten einer "trockenen" und faltigen Haut, die insb. häufig, aber nicht ausschließlich, im höheren Lebensalter auftritt.

Ein bekanntes Beispiel für derartige Veränderungen ist die Verarmung der Lipid-Doppelschichten des Stratum corneum der menschlichen Haut an Ceramiden. Beispielsweise zeigten sich im Fall der Atopischen Dermatitis in der Haut der Patienten erniedrigte Gehalte u.a. an Ceramid 3, Ceramid 4, ω-Hydroxyceramiden (Macheleidt O, Kaiser HW und Sandhoff K (2002): J Invest Dermatol 119(1): 166-173) und Sphingosin.

Wie zahlreiche weitere wissenschaftliche Publikationen der vergangenen Jahre zeigen, basieren verschiedene Hautveränderungen und Hauterkrankungen auf den Veränderungen nicht nur der Ceramid- sondern der gesamten Lipid-Zusammensetzung der Stratum-corneum-Schicht der menschlichen Haut. Diese Veränderungen führen zu einem mehr oder weniger stark erniedrigten Wasserbindevermögen und zu einer eingeschränkten Barrierefunktion der betroffenen Hautpartien für Wasser, d.h. zu einer Erhöhung des TEWL-Werts (McIntosh TJ, Stewart ME, Downing DT (1996): Biochemistry 35(12) : 3.649-3.653; Coderch L, de Pera M, Perez-Cullell N, Estelrich J, de la Maza A, Parra JL (1999): Skin Pharmacol Appl Skin Physiol 12(5) : 235-246). Hautveränderungen und Hauterkrankungen dieser Art sind beispielsweise:
- Atopische Dermatitis
- "trockene" Haut-Xerose, Xerodermie
- dyshidrotisches Ekzem
- chronisches kumulativ-toxisches Kontaktekzem
- alternde Haut
- durch UV-Licht stark beanspruchte Haut
- Sebostase
- Verhornungsstörungen
- Diabetes-bedingte Hautschäden

Insbesondere auf dem Gebiet der Klinischen Medizin ist es in den genannten Fällen von Erkrankungen wünschenswert, die Struktur der im Organismus vorhandenen biologischen Membran, i. e. der Lipid-Doppelschichten, in geeigneter Weise zu verändern und/oder zu stabilisieren.

Gemäß neueren Erkenntnissen zum Pathomechanismus der Atopischen Dermatitis (Arikawa J, Ishibashi M, Kawashima M, Takagi Y, Ichikawa Y, Imokawa G (2002): J Invest Dermatol 119(2): 433-439) und verwandter Erkrankungen ist die Ursache für die Anfälligkeit der Haut im Fall einer derartigen Erkrankung u. a. ein veränderter Lipidstoffwechsel bzw. reduzierter Lipid-Gehalt des Stratum corneum. Diese Veränderungen betreffen neben dem Ceramid-Stoffwechsel u.a. den Fettsäure-Stoffwechsel. So zeigten sich bei der Atopischen Dermatitis, aber auch bei anderen Hauterkrankungen wie bei Psoriasis, bei (lamellarer) Ichthyosis und bei Kontaktdermatitis in der Haut der Patienten erniedrigte Gehalte an freien Fettsäuren (Pilgram GS, Vissers DC, van der Meulen H, Pavel S, Lavrijsen SP, Bouwstra JA, Koerten HK (2001): J Invest Dermatol 117(3): 710-717); Man MQM, Feingold KR, Thornfeldt CR, Elias PM (1996): J Invest Dermatol 106(5): 1096-1101; Mao-Qiang M, Elias PM, Feingold KR (1993): J Clin Invest 92: 791-798; Mao-Qiang M, Jain M, Feingold KR, Elias PM (1996): J Invest Dermatol 106(1): 57-63; Velkova V, Lafleur M (2002): Chem Phys Lipids 117(1-2): 63-74.

Die heutigen Möglichkeiten, die Symptome und Folgen der genannten Hautkrankheiten, insb. der Atopischen Dermatitis, zu lindern (von einer Heilung kann derzeit noch keine Rede sein), sind nach wie vor noch sehr begrenzt. Die topische Anwendung spezieller Glucocorticoide und immunsuppressiver Wirkstoffe ist wegen der Toxizität dieser Substanzen mit erheblichen Risiken verbunden. Bestimmte Corticoide verursachen sogar einen geradezu kontraproduktiven Effekt, indem sie zu einem Verlust an Lipiden, insb. an Ceramiden, Cholesterol und freien Fettsäuren führen.

Unter Berücksichtigung des heutigen Kenntnisstands über die Bedeutung einer physiologischen Lipid-Zusammensetzung der Stratum-corneum-Membranen ist es folgerichtig, dass versucht wird, die im Stratum corneum vorhandenen Defizite an Membran-Lipiden durch exogene Zufuhr auszugleichen. In der Praxis versucht man daher, mit Hilfe von Salben, Cremes und dergleichen die fehlenden Lipide, z.B. Ceramide und Freie Fettsäuren, der veränderten bzw. erkrankten Haut zuzuführen. Dies erfolgt beispielsweise durch speziell hierfür formulierte Lipid-Präparate, u. a. unter Verwendung von Liposomen als Vehikel zum Transport der Lipide in die Haut. Zahlreiche Produkte sind zu kosmetischen Zwecken und zur Therapie der genannten Hautkrankheiten inzwischen auf dem Markt.

Die hier geschilderten therapeutischen Maßnahmen sind sicherlich als prinzipiell richtig anzusehen, da sie in logischer Weise die vorhandenen Defizite des Stratum corneum an Lipiden und Lipid-analogen Substanzen auszugleichen versuchen. Die während der letzten Jahre mit diesen therapeutischen Maßnahmen gemachten Erfahrungen zeigen jedoch, dass trotz der prinzipiellen Richtigkeit des therapeutischen Ansatzes die Ergebnisse dieser kosmetischen und medizinischen Behandlungen keineswegs überzeugend sind. Zum Teil ist der Erfolg der durchgeführten Maßnahmen unsicher bzw. nicht nachhaltig. Selbst dann, wenn sich ein annähernd akzeptabler Erfolg der Heilbehandlung einstellt, hat eine derartige Behandlung zumindest zwei gravierende Nachteile:
- Das Ausmaß des Heilerfolgs ist nicht so groß, dass von einer vollständigen Gesundung der erkrankten Haut gesprochen werden kann.
- Um einen einigermaßen akzeptablen Heilerfolg an der Haut über einen längeren Zeitraum zu gewährleisten, müssen die genannten Lipide und Lipid-analogen Substanzen in kurzen Zeitabständen permanent der Haut zugeführt werden, d.h. die eingesetzten Wirksubstanzen zeigen keine nachhaltige Wirksamkeit.

Beide Nachteile sind auf eine gemeinsame Ursache zurückzuführen. Die genannten Lipide sind keine statischen Komponenten der Haut, sondern sie sind Zwischenprodukte einer Reaktionsfolge, in der die von der Haut benötigten Lipide z.B. durch Synthesevorgänge des Organismus oder aus der Nahrung bereitgestellt und in die biologische Membran eingebaut werden. Nach Wahrnehmung ihrer Funktion als Membranbestandteil des Stratum corneum werden sie anschließend in spezifische Abbaureaktionen des Organismus eingeschleust.

Diese Reaktionsabfolge stellt ein Fließgleichgewicht dar, in welchem eine gewisse Menge der genannten Komponenten durch die Wirkung bestimmter Enzyme synthetisiert und nach einer bestimmten Verweildauer in der Membran aus dieser wieder freigesetzt wird, um dann über enzymgesteuerte metabolische Abbauprozesse eliminiert zu werden. Es liegt somit ein gewisser Durchsatz an Substanz vor. Die als Hauttherapeutica exogen zugeführten Lipide werden in diese Reaktionsabfolge eingeschleust. Liegt *a priori* eine Störung dieser Reaktionsabfolge vor, die dann zu einer pathologisch verminderten Lipid-Zusammensetzung des Stratum corneum führt, so ist zu erwarten, dass die exogene Zufuhr von Lipiden in Form eines Therapeuticums an diesem pathologischen Zustand grundlegend nichts oder nicht viel ändern kann, da der exogen zugeführte Lipid-Anteil vom Organismus in gleicher Weise metabolisiert wird, wie dies bei dem endogen vorhandenen Lipid-Anteil der Fall ist.

Ein Heilerfolg ist daher mit den derzeit zur Verfügung stehenden therapeutischen Möglichkeiten in hohem Maße davon abhängig, dass die betreffenden therapeutischen Ersatzstoffe schneller in die Haut eindringen können als sie in die vorhandenen physiologischen Abbauschritte eingeschleust werden, und dass sie über einen längeren Zeitraum, im Extremfall lebenslang, kontinuierlich zugeführt werden.

Das vorliegende Problem ist nicht ohne weiteres lösbar. Der Geschwindigkeit der Aufnahme von Lipiden und Lipid-analogen Substanzen in das Stratum corneum hinein sind gewisse physiologische und physikalischchemische bzw. biochemische Grenzen gesetzt, z. B. hinsichtlich der Diffusionsgeschwindigkeit der kosmetischen und therapeutischen Wirksubstanzen. Diese Geschwindigkeit kann nicht beliebig gesteigert werden. Zum anderen sind die an den genannten Reaktionsfolgen beteiligten Lipid-synthetisierenden und Lipidabbauenden Enzyme durch exogene Maßnahmen nicht oder nicht ohne gravierende Probleme im Sinne einer Erhöhung (synthetisierende Enzyme) oder einer Minderung ihrer Aktivität(metabolisierende Enzyme) zu beeinflussen.

Der erstgenannte therapeutische Ansatz, i.e., die Aktivierung lipidsynthetisierender Enzyme durch exogene Wirkstoffe (z.B. Nicotinamid), ist nur in begrenztem Umfang möglich und bisher lediglich *in vitro* gelungen (Tanno O, Ota Y, Kitamura N, Katsube T, Inoue S (2000): British J Dermatol 143(3): 524-531). Der zweite therapeutische Ansatz, i.e., die Hemmung Lipid-metabolisierender Enzyme durch exogene Wirkstoffe ist bisher offenbar noch nicht gelungen, da offensichtlich Hemmstoffe Lipid-metabolisierender Enzyme mit ausreichend hoher Spezifität nicht existieren.

Zur Lösung des beschriebenen Problems ist es erforderlich, grundsätzlich andere Prinzipien anzuwenden, um die therapeutische Wirksamkeit exogen zugeführter Lipid-Ersatzsubstanzen oder -Analogsubstanzen zu erhöhen.

Die Aufgabe der vorliegenden Erfindung ist es deshalb, Verbindungen bereitzustellen, durch welche
- die im Organismus vorhandenen biologischen Membranen stabilisiert werden können,
- die Wasserbindefähigkeit der verschiedenen Hautschichten erhöht werden kann und
- der transepidermale Wasserverlust (TEWL = Trans Epidermal Water Loss) der Haut vermindert werden kann.

Unter der Stabilisierung der biologischen Membran soll im vorliegenden Fall der Vorgang verstanden werden, dass die erfindungsgemäßen Wirksubstanzen nach Einlagerung in die biologische Membran eine gegenüber den unveränderten originären Lipiden geringere Tendenz zum Verlassen der Membran zeigen, d.h. dass sie eine größere Nachhaltigkeit ihrer kosmetischen und klinischen Wirksamkeit besitzen.

Diese Aufgabe wird durch die Lipid-Hybridmoleküle mit den Merkmalen des Anspruchs 1, hinsichtlich der Verwendung als Arzneimittel mit den Merkmalen des Anspruchs 12 oder 13 sowie hinsichtlich der Verwendung als kosmetische Zubereitung mit den Merkmalen des Anspruchs 15 gelöst. Die weiteren abhängigen Ansprüche zeigen vorteilhafte Weiterbildungen auf.

Erfindungsgemäß werden Hybridmoleküle aus zwei Lipiden oder Lipid-analogen Verbindungen ausgewählt aus der Gruppe bestehend aus Ceramiden, Sphingosinen, Phospholipiden, Glykolipiden, Fettsäuren, Sterolen und deren Kombinationen bereitgestellt, wobei die Lipide oder Lipid-analogen Verbindungen über ihr lipophiles Ende miteinander verknüpft sind und am hydrophilen Ende der Lipide oder Lipid-analogen Verbindungen mindestens eine hydrophile Gruppe zur Vergrößerung der Hydrathülle des Hybridmoleküls angeordnet ist.

Die erfindungsgemäßen Verbindungen weisen folgende Eigenschaften auf:
- Die grundsätzliche Struktur der eingesetzten Lipide oder Lipid-analogen Substanzen, welche die Ausbildung der Lipid-Doppelmembran ermöglicht, bleibt nicht nur erhalten, sondern die Fähigkeit zur Ausbildung der Doppelmembran wird verstärkt, weil die durch die genannten Erkrankungen geschädigte Haut ohnehin nur eine eingeschränkte Fähigkeit zum Aufbau und zum Erhalt der physiologischen Lipid-Doppelmembran besitzt.
- Die Struktur der eingesetzten Lipide oder Lipid-analogen Substanzen wird bei erhaltener Grundstruktur so verändert, dass sie nur noch in geringerem Umfang als Substrate der in der Haut, speziell im Stratum corneum, vorhandenen metabolisierenden Enzyme fungieren können. Dies bedeutet, dass sie in deutlich geringerem Umfang als die originären Lipide oder Lipid-analogen Substanzen in die jeweiligen enzymatischen Reaktionsfolgen eingeschleust werden und somit als wesentliche strukturelle Bestandteile des Stratum corneum über einen längeren Zeitraum als die originären Lipide bzw. Lipid-analogen Substanzen erhalten bleiben.
- Die Abänderung der Molekülstruktur erfolgt jedoch andererseits nur in einem so geringen Umfang, dass durch den geringen stoffwechselbedingten Um- oder Abbau der zugeführten Lipid-Hybridmoleküle solche Substanzen entstehen, die den körpereigenen Lipiden oder Lipid-analogen Substanzen möglichst ähnlich sind. Auf diese Weise wird die Gefahr erheblich verringert, dass Stoffwechselprodukte mit toxischer Wirkung entstehen.
- Eine vergleichsweise geringe, steuerbare Abbaubarkeit der Lipid-Hybridmoleküle wird dadurch erreicht, dass die kovalente Bindung zwischen den beiden originären Lipidmolekülen über ein Sauerstoffatom erfolgt. Dieses Sauerstoffatom wirkt als metabolische Sollbruchstelle, da z.B. Cytochrom-P₄₅₀-abhängige mischfunktionelle Monooxygenasen die in unmittelbarer Nachbarschaft zum Sauerstoffatom stehenden Kohlenstoffatome oxidativ angreifen, was zu einem Auseinanderbrechen des Hybridmoleküls unter Bildung der ω-hydroxylierten originären Lipide führt.
- Durch die Anbindung eines weiteren Moleküls mit ausgeprägten hydrophilen Eigenschaften an den hydrophilen äußeren Molekülteilen der Lipid-Hybridmoleküle wird die Hydrathülle der Wirksubstanzen erhöht, was in der Folge zur Erhöhung der Wasserbindefähigkeit der betreffenden Hautschichten und zu einer Verminderung des transepidermalen Wasserverlustes (TEWL) führt.

Diese Eigenschaften werden erfindungsgemäß dabei folgendermaßen realisiert:

Durch die Kopplung zweier Lipidmoleküle zu Lipid-Hybridmolekülen ist die Gewähr gegeben, dass ein derartiges Molekül durch die im Organismus vorhandenen Enzyme des Lipid-Stoffwechsels sehr viel langsamer ab- bzw. umgebaut wird, als dies für die originären Lipide zutrifft. Die mit der kovalenten Bindung zwischen zwei Lipidmolekülen verbundene Molekülvergrößerung führt zu einer starken Minderung der enzymatisch gesteuerten Metabolisierung, weil bei der bekanntermaßen hohen Substratspezifität der meisten Enzyme die (annähernde) Verdopplung der Größe des Lipidmoleküls die Geschwindigkeit des Lipidumsatzes bzw. des Lipidabbaus erheblich abfallen lässt.

Andererseits sind die entstehenden Abbauprodukte von ihrem allgemeinen Aufbau her den natürlicherweise vorkommenden Folgeprodukten des Lipid-Stoffwechsels so ähnlich, dass ein Einschleusen in die entsprechenden Reaktionsfolgen nach einem langsam ablaufenden Abbau der erfindungsgemäßen Wirksubstanzen ohne Probleme möglich ist. Darüber hinaus ist auch in keiner Weise damit zu rechnen, dass die Lipid-Hybridmoleküle wegen der großen Ähnlichkeit mit den originären Lipid-Molekülen eine relevante Toxizität besitzen.

Ein gewisser Grad der enzymatischen Metabolisierung der Lipid-Hybridmoleküle, die allerdings als deutlich geringer anzusehen ist als die der monomeren Lipid-Moleküle, ist somit eine aus pharmakokinetischen und pharmakodynamischen Gründen erwünschte Eigenschaft des Moleküls, weil hierdurch die Steuerbarkeit der Therapie besser gewährleistet ist, als wenn kein metabolischer Abbau möglich wäre.

Eine gewisse kontrollierte Metabolisierungsgeschwindigkeit der Lipid-Hybridmoleküle lässt sich dadurch erreichen, dass zwischen den beiden aneinandergekoppelten Lipidmolekülen ein Sauerstoffatom vorhanden ist. Die in Nachbarschaft zu diesem Sauerstoffatom stehenden Kohlenstoffatome können enzymatisch hydroxyliert werden, etwa durch die Cytochrom-P₄₅₀-abhängigen mischfunktionellen Monooxygenasen. Eine derartige, in unmittelbarer Nachbarschaft zum Sauerstoffatom stattfindende Hydroxylierung führt zur Bildung instabiler Verbindungen mit Halbacetalstruktur, die in die entsprechenden Reaktionsprodukte zerfallen. Das eine Reaktionsprodukt ist ein Lipidmolekül mit ω-ständiger OH-Gruppe, das andere Reaktionsprodukt ist ein Lipidmolekül mit ω-ständiger Aldehydfunktion, die zur Carbonsäuregruppe weiteroxidiert wird. Damit wird offensichtlich, dass durch einen oxidativ ablaufenden biochemischen Abbau der beschriebenen Lipid-Hybridmoleküle Reaktionsprodukte entstehen, die den Ausgangsverbindungen der originären Lipidmoleküle sehr ähnlich sind.

Ein weiterer wesentlicher Aspekt der Pathogenese der oben genannten Hautveränderungen bzw. Hauterkrankungen ist die reduzierte Wasserbindefähigkeit des Hautgewebes, insbesondere im Bereich des Stratum corneum. Physiologischerweise wird das Wasser nicht innerhalb, sondern, da mehrere parallel angeordnete LipidSchichten vorliegen, in den Raum zwischen den einzelnen Lipid-Doppelschichten eingelagert. Dies liegt in der Tatsache begründet, dass das Innere der Lipid-Doppelschicht aus den stark hydrophoben Fettsäureresten aufgebaut ist, während das Medium außerhalb der Lipid-Doppelschicht hydrophiler Natur ist. Eine Speicherung von Wasser in den hydrophoben inneren Bereichen der Lipid-Doppelmembran ist praktisch nicht möglich.

Die anfangs genannten Hautveränderungen und -erkrankungen sind zum einen auf den Verlust eines Teiles der Lipide aus den parallel angeordneten Lipid-Doppelschichten, zum anderen auf den partiellen Verlust von Wasser aus den zwischen diesen Doppelschichten angeordneten hydrophilen Zwischenschichten zurückzuführen. Ziel der therapeutischen Maßnahmen bei diesen Erkrankungen ist somit nicht nur der Wiederaufbau und die Stabilisierung der Lipid-Doppelschichten selbst, wie dies mit Hilfe der oben beschriebenen Lipid-Hybridmoleküle erfolgt, sondern weiterhin auch der Aufbau und die Stabilisierung einer ausgeprägten Hydrosphäre an der Oberfläche der Lipid-Doppelschicht, die für die Wasserbindefähigkeit der Haut von ausschlaggebender Bedeutung sind.

Die Lipide aus der Gruppe der Ceramide bestehen dabei bevorzugt aus den bisher in der wissenschaftlichen Literatur beschriebenen Verbindungen Ceramid-1 (Ceramid EOS), Ceramid-2 (Ceramid NS), Ceramid-3 (Ceramid NP), Ceramid-4 (Ceramid EOH), Ceramid-5 (Ceramid AS), Ceramid-6 (Ceramid AP), Ceramid-7 (Ceramid AH), Ceramid-8 (Ceramid NH) und Ceramid-9 (Ceramid EOP) sowie den entsprechenden ω-Hydroxyceramiden. Die genannten Ceramide stellen allerdings keine einheitlichen Substanzen mit genau definierter relativer Molmasse dar, sondern jedes Ceramid stellt eine Familie von Verbindungen mit unterschiedlicher Länge der im Molekül vorhandenen amidartig gebundenen Fettsäure und/oder des im Sphingosinanteil vorhandenen Alkylrestes dar.

Die Lipide aus der Gruppe der Sphingosinderivate sind bevorzugt das Sphingosin selbst und das Sphingomyelin, die strukturelle Ähnlichkeit mit den Ceramiden besitzen.

Die Lipide aus der Gruppe der Phospholipide sind bevorzugt das Phosphatidylserin, Phosphatidylethanolamin, Phosphatidylcholin sowie Phosphatidylinositol.

Die Lipide aus der Gruppe der Glykolipide sind bevorzugt die Cerebroside mit dem Sphinosin-Grundgerüst und einem Zuckerrest (Glucose oder Galaktose) sowie die komplexen Glykolipide mit bis zu sieben Zuckerresten, die als Ganglioside bezeichnet werden.

Die Lipid-analogen Substanzen aus der Gruppe der Fettsäuren bestehen bevorzugt aus Palmitinsäure, Stearinsäure oder Oleinsäure oder aus anderen gesättigten oder ungesättigten Monocarbonsäuren mit einer Kettenlänge zwischen 10 und 40 C-Atomen. Bevorzugt sind die Fettsäuren ausgewählt aus der Gruppe bestehend aus n-Hexadecansäure (Palmitinsäure, C₁₅H₃₁-COOH), n-Dodecansäure (Laurinsäure, C₁₁H₂₃-COOH), n-Tetradecansäure (Myristicinsäure, C₁₃H₂₇-COOH), n-Octadecansäure (Stearinsäure, C₁₇H₃₅-COOH), n-Eicosansäure (Arachinsäure, C₁₉H₃₉-COOH), n-Tetracosansäure (Lignocerinsäure, C₂₃H₄₇-COOH), 9-Hexadecensäure (Palmitoleinsäure, C₁₅H₂₉-COOH), 9-Octadecensäure (Oleinsäure, Ölsäure, C₁₇H₃₃-COOH), 9,11-Octadecadiensäure (C₁₇H₃₁-COOH), 9,12-Octadecadiensäure (Linolsäure, C₁₇H₃₁-COOH), 9,12,15-Octadecatriensäure (Linolensäure, C₁₇H₂₉-COOH), 5,8,11,14,17-Eicosapentaensäure ("EPA", C₁₉H₂₉-COOH), 4,7,10,13,16,19-Docosahexaensäure ("DHA", C₂₁H₃₁-COOH), Decansäure (C₁₀H₂₁-COOH), Octacosansäure (C₂₈H₅₇-COOH) und 9-Octacosensäure (C₂₈H₅₅-COOH) .

Die Lipid-analogen Substanzen aus der Gruppe der Sterole sind bevorzugt das Cholesterol, Cholesterolsulfat und verschiedene Cholesterolester sowie weitere Sterole wie Lanosterol und Sitosterol.

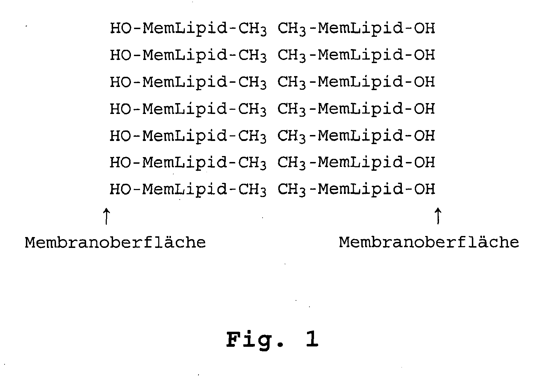

Biologische Membran sind ihrer Struktur nach Lipiddoppelmembranen, in denen die lipophilen Bereiche der membranbildenden Lipide, u.a. auch der Ceramide, im Innern der Membran angeordnet sind, wodurch die folgende allgemeine Struktur entsteht, bei die hydrophilen Bereiche der Moleküle an der Membranoberfläche dem Extra- bzw. dem Intrazellularraum zugewandt sind. In Fig. 1 wird die Anordnung der membranbildenden Lipide (als "HO-MemLipid-CH₃" bezeichnet) dargestellt.

In der in Fig. 1 angegebenen allgemeinen Struktur der Membranlipide

HO-MemLipid-CH₃

kennzeichnet die OH-Gruppe den hydrophilen Bereich des jeweiligen Lipids, z.B. die in der Sphingosinstruktur der Ceramide enthaltenen OH-Gruppen oder die in 3-Position des Cholesterolmoleküls stehende OH-Gruppe oder die OH-Gruppe in der Carboxylfunktion der Fettsäuren (wie z.B. Palmitinsäure). Die angegebene CH₃-Gruppe kennzeichnet den hydrophoben Bereich des betreffenden Membranlipids, z.B. den Alkan- oder Alken-Rest der amidartig gebundenen Fettsäure in den Ceramiden, die verzweigte Alkylseitenkette des Cholesterol-Moleküls oder den Alkan- oder Alkenrest einer Fettsäure.

Zwischen den lipophilen Bereichen der Lipidmoleküle wirken relativ schwache molekulare Anziehungskräfte, sog. Van-der-Waals-Kräfte. Die Kräfte sind genügend groß, um der Membran eine gewisse Stabilität verleihen, was u.a. daran zu erkennen ist, dass sich die Struktur der Membran spontan ausbildet. Die Kräfte sind andererseits jedoch nicht so groß, dass sie einem (insb. krankheitsbedingten) teilweisen Verlust von Lipiden aus der Membran entgegenwirken könnten.

Der Verlust von Lipiden aus der Membran kann jedoch dann wirksam vermindert oder verhindert werden, wenn zwischen den lipophilen Bereichen bestimmter Lipidmoleküle nicht nur Van-der-Waals-Kräfte wirken, sondern wenn sie durch eine kovalente Bindung verknüpft sind, wie es im nächstfolgenden Schema 2 angegeben ist. Dieses Schema beschreibt eine Lipid-Doppelmembran, in die Membranlipide ("MemLipid") eingelagert sind, die jeweils zwischen den beiden lipophilen Enden der Lipid-Moleküle eine kovalente Bindung aufweisen. Aus der schematischen Darstellung einer derartigen Verknüpfung

HO-MemLipid-CH₂-CH₂-MemLipid-OH

geht hervor, dass die Verknüpfung zweier identischer Lipidmoleküle zum Dimer oder zweier unterschiedlicher Lipidmoleküle zum sog. Lipid-Hybridmolekül über die beiden endständigen CH₃-Gruppen der jeweiligen Lipid-Monomere erfolgt. Die beiden OH-Gruppen der resultierenden Verbindung kennzeichnen die beiden hydrophilen Bereiche an den Enden des Dimers oder des Lipid-Hybridmoleküls.

Wegen der unter Punkt 4 der oben angegebenen Anforderungen erwünschten (geringen) Metabolisierbarkeit empfiehlt es sich, zwischen die beiden zu verbindenden Lipid-Moleküle ein Sauerstoffatom einzufügen. Damit ergibt sich die folgende Struktur:

HO-MemLipid-CH₂-O-CH₂-MemLipid-OH

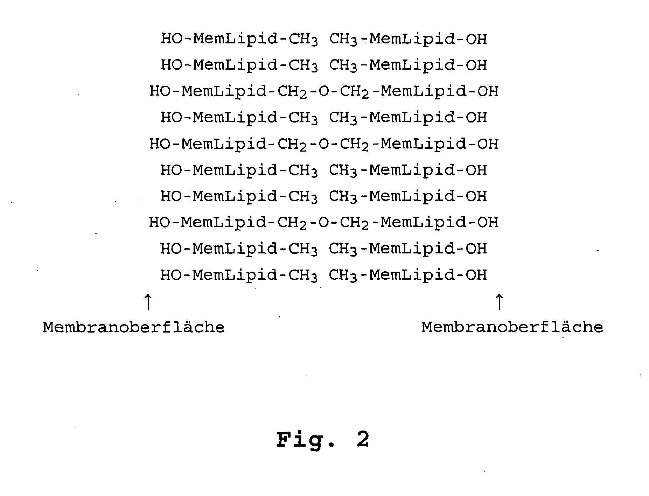

Die Einlagerung solcher Lipid-Hybridmoleküle in die Lipiddoppelmembran ergibt die in Fig. 2 gezeigte Struktur.

Durch die kovalente Bindung innerhalb der nun vorliegenden Lipid-Hybridmoleküle nimmt die Stabilität dieser Membranstruktur gegenüber der normalen biologischen Membranstruktur erheblich zu.

Bei der alternden Haut und verschiedenen Hauterkrankungen tritt jedoch nicht nur ein partieller Lipidverlust der in der Haut vorhandenen Lipidschichten auf, sondern es findet sich auch eine erniedrigte Wasserbindefähigkeit bzw. ein erhöhter transepidermaler Wasserverlust (TEWL) verschiedener Hautschichten.

Zwar wird eine gewisse Menge Wasser an den zum Intra- und Extrazellularraum gerichteten hydrophilen Molekülteilen der membranständigen Lipide gebunden und trägt somit zum natürlichen Wasserreservoir der Haut bei. Bei der alternden oder erkrankten Haut reicht diese Wasserbindefähigkeit jedoch offensichtlich nicht mehr aus, um die natürliche Struktur der Haut zu erhalten. Es ist daher erforderlich, diese Wasserbindungskapazität der Hautmembranen dadurch zu erhöhen, dass an den hydrophilen Molekülteilen der Membranlipide zusätzlich hydrophile Moleküle angekoppelt werden. Da, wie die Bezeichnung schon sagt, hydrophile Moleküle eine besonders hohe Affinität zu Wasser haben, führt die Ankopplung solcher Moleküle zu einer Vergrößerung der Hydrathülle der Membran.

Hydrophile Gruppen mit einer gesteigerten Tendenz zur Bindung von Wasser sind stark polare Verbindungen, insb. mit positiven oder negativen Ladungen und/oder mit solchen funktionellen Gruppen, die Wasser über Wasserstoffbrückenbindungen anzulagern vermögen. Hierzu gehören insbesondere

Aminosäuren, die stark polare Gruppen oder Ladungen besitzen: -COOH, -NH₂, -COO⁻ und -NH₃⁺, bevorzugt Serin, Threonin, Lysin, Arginin, Histidin, Asparaginsäure, Glutaminsäure, Asparagin, Glutamin, Tyrosin und Tryptophan.

Polyole, wie Ethandiol oder Glycerin (Propantriol) mit mehreren -OH-Gruppen im Molekül, die zur Ausbildung von Wasserstoffbrückenbindungen befähigt sind.

Zucker wie Glucose oder Galaktose, bei denen mehrere OH-Gruppen im Molekül vorhanden sind.

Zucker-Derivate, wie Glucuronsäure oder Galakturonsäure mit mehreren OH-Gruppen und einer dissoziierbaren -COOH-Gruppe Zucker-Derivate, wie Aminozucker, die Bestandteile der stark wasserbindenden Hyaluronsäure darstellen.

Organische Säuren, wie die Di- bzw. Tricarbonsäuren Malonsäure, Bernsteinsäure, Äpfelsäure oder Citronensäure, deren nicht für die Bindung an das Lipidmolekül gebrauchten weiteren Carboxylreste dissoziiert und daher geladen vorliegen, was mit einer hohen Wasserbindefähigkeit einhergeht.

Anorganische Säuren, wie z.B. Sulfat und Phosphat, die auf Grund ihrer im Molekül vorhandenen Ladungen eine sehr hohe Wasserbindefähigkeit besitzen. Cholesterolsulfat kommt als natürlicher Bestandteil biologischer Membranen vor.

Cholin als physiologische Substanz mit einem (positiv geladenen) quaternären N-Atom.

Derivate des Harnstoffs: Diese Verbindung (auch im Deutschen in kosmetischen und medizinischen Präparaten unter der englischen Bezeichnung "urea" geführt) ist in freier Form bereits heute Bestandteil vieler Salben, mit denen eine Erhöhung der Wasserbindefähigkeit der Haut bewirkt werden soll.

Die Lipid-Moleküle sind vorzugsweise über ihr lipophiles Ende kovalent miteinander verbunden. Dabei können die Lipid-Moleküle auch über einen Spacer verbunden sein. Der Spacer besteht dabei vorzugsweise aus mindestens einem Atom ausgewählt aus der Gruppe bestehend aus Kohlenstoff, Stickstoff oder Sauerstoff oder Kombinationen hiervon, vorzugsweise bestehend aus einem Sauerstoffatom oder einer -O-(CH₂)ₙ-O-Gruppe mit n = 1 bis 20. Ebenso ist es möglich, dass der Spacer eine der genannten Gruppen enthält.

Erfindungsgemäß wird ebenso das zuvor beschriebene Dimer oder das Lipid-Hybridmolekül zur Verwendung als Arzneimittel bereitgestellt.

Erfindungsgemäß wird das Dimer oder das Lipid-Hybridmolekül, wie beide zuvor beschrieben wurden, zur Herstellung eines Arzneimittels zur Behandlung von Erkrankungen, bei denen eine Störung der Lipidzusammensetzung der Zellmembranen eines Organismus hinsichtlich ihres Gehalts an Lipiden oder Lipid-analogen Substanzen vorliegt, bereitgestellt.

Das zuvor beschriebene Dimer oder das Lipid-Hybridmolekül kann auch zur Herstellung eines Arzneimittels zur Behandlung von Erkrankungen, bei denen eine Störung der Zusammensetzung der Lipid-Doppelschichten des Stratum corneum der Haut hinsichtlich ihres Gehaltes an Lipiden oder Lipid-analogen Substanzen vorliegt, eingesetzt werden.

Eine weitere Verwendung der erfindungsgemäßen Dimere betrifft die Herstellung von kosmetischen Zubereitungen, insbesondere als Creme, Salbe, Lotion, Emulsion, Gel, Spray, kosmetisches Öl oder Liposomen.

Wird erfindungsgemäß an Hydroxylgruppen der hydrophilen Bereiche eines Lipid-Hybridmoleküls zum Beispiel jeweils ein Serin-Rest angekoppelt, so ergibt sich die folgende Struktur:

Serin-O-MemLipid-CH₂-O-CH₂-MemLipid-O-Serin

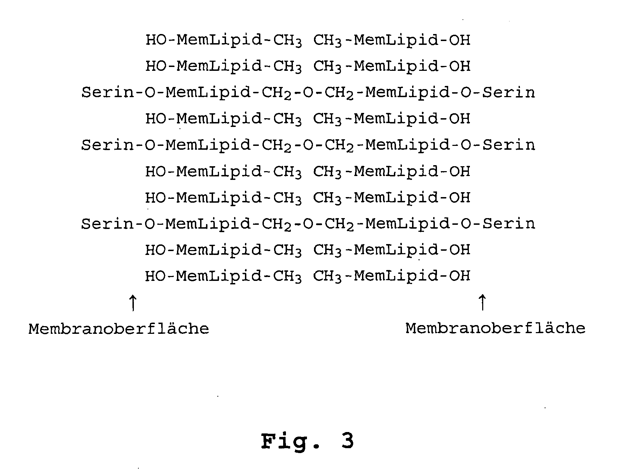

Die Einlagerung eines derartig modifizierten Lipid-Hybridmoleküls in die Lipidmembran (Fig. 3) führt zu einer deutlichen Erhöhung der Hydrophilie der Membranoberfläche und damit zu einer Erhöhung der Wasserbindefähigkeit der Membran und in der Folge auch der Haut.

Anhand der nachfolgenden Figuren und Beispiele soll der erfindungsgemäße Gegenstand näher erläutert werden, ohne diesen auf die hier gezeigten speziellen Ausführungsformen einschränken zu wollen.
- Fig. 1: zeigt den prinzipiellen Aufbau einer biologi- schen Membran als Doppelschicht aus Membranli- piden.
- Fig. 2: zeigt schematisch den Aufbau einer biologi- schen Membran aus Membranlipid-Molekülen und Lipid-Hybridmolekülen aus zwei Membranlipid- Komponenten, die über eine Sauerstoffbrücke kovalent verbunden sind.
- Fig. 3: zeigt schematisch den Aufbau einer biologi- schen Membran aus Membranlipid-Molekülen mit der zusätzlichen Einlagerung von Lipid- Hybridmolekülen mit zwei angekoppelten Serin- Resten.
- Fig. 4: beschreibt eine Wirksubstanz, in der das Li- pid-Hybridmolekül aus Ceramid 2 und Choleste- rol besteht. Zur Erhöhung der Hydrophilie der an der Membranoberfläche liegenden hydrophilen Molekülareale ist am Ceramid 2 ein Serinrest, am Cholesterol ein Sulfatrest angekoppelt.
- Fig. 5: beschreibt eine Wirksubstanz, in der das Li- pid-Hybridmolekül aus Ceramid 3 und Palmitin- säure besteht. Zur Erhöhung der Hydrophilie der an der Membranoberfläche liegenden hydro- philen Molekülareale ist am Ceramid 3 ein Ly- sinrest, an der Palmitinsäure ein Harnstoff- Rest angekoppelt.
- Fig. 6: beschreibt eine Wirksubstanz, in der das Li- pid-Hybridmolekül aus Linolsäure und Choleste- rol besteht. Zur Erhöhung der Hydrophilie der an der Membranoberfläche liegenden hydrophilen Molekülareale ist an der Linolsäure ein Gluco- serest, am Cholesterol ein Asparaginsäurerest angekoppelt.

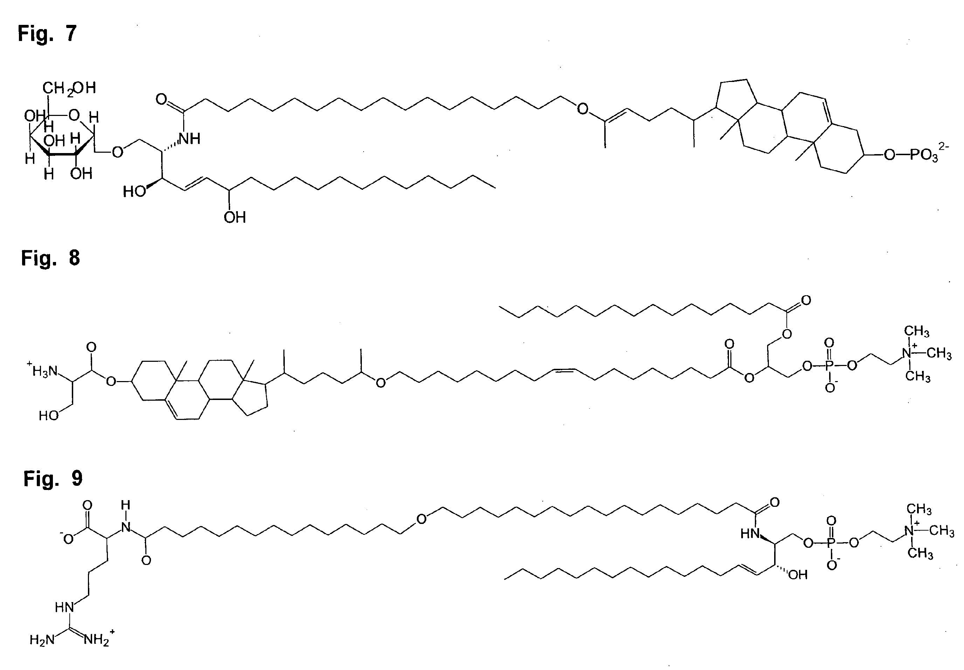

- Fig. 7: beschreibt eine Wirksubstanz, in der das Li- pid-Hybridmolekül aus Ceramid 8 und Lanosterol besteht. Zur Erhöhung der Hydrophilie der an der Membranoberfläche liegenden hydrophilen Molekülareale ist am Ceramid 8 ein Galaktose- rest, am Lanosterol ein Phosphatrest angekop- pelt.
- Fig. 8: beschreibt eine Wirksubstanz, in der das Li- pid-Hybridmolekül aus Cholesterol und Phospha- tidylcholin besteht. Zur Erhöhung der Hydro- philie der an der Membranoberfläche liegenden hydrophilen Molekülareale ist am Cholesterol ein Serinrest angekoppelt, während das Phosphatidylcholin wegen seines schon vorhan- denen ausgeprägt hydrophilen Molekülteils un- verändert bleibt.
- Fig. 9: beschreibt eine Wirksubstanz, in der das Li- pid-Hybridmolekül aus Palmitinsäure und Sphin- gomyelin besteht. Zur Erhöhung der Hydrophilie der an der Membranoberfläche liegenden hydro- philen Molekülareale ist an der Fettsäure ein Argininrest angekoppelt, während das Sphingo- myelin wegen seines schon vorhandenen ausge- prägt hydrophilen Molekülteils unverändert bleibt.

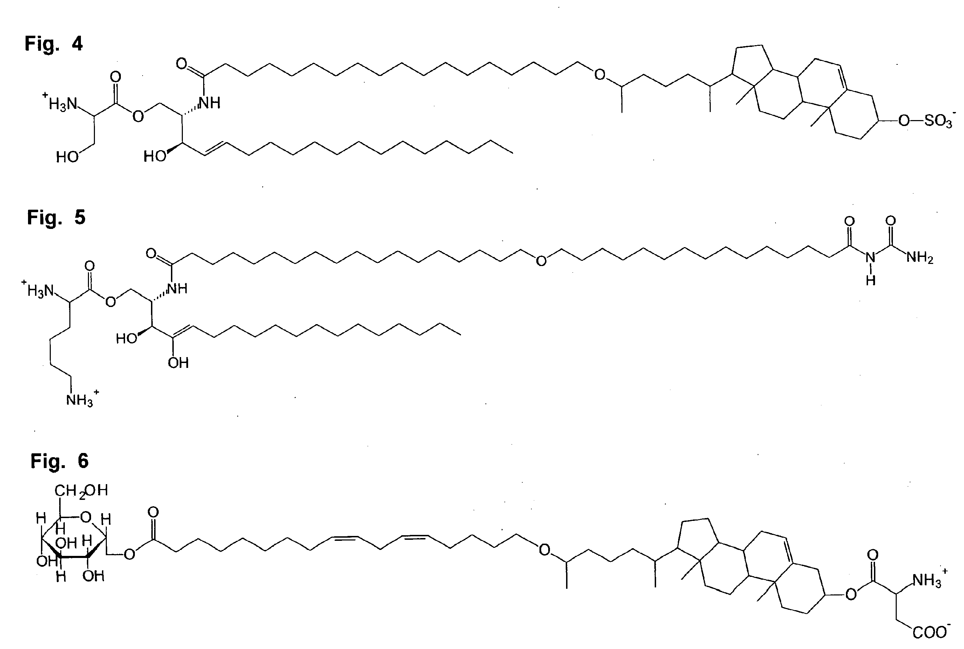

Der Aufbau einer Harnstoff-ähnlichen Struktur (s. Fig. 5) ist deswegen von besonderem Interesse, weil Harnstoff über ein sehr hohes Wasserbindevermögen verfügt, was heute bereits in Form Harnstoff-haltiger Salben zur kosmetischen und therapeutischen Behandlung von solchen Hauterkrankungen genutzt wird, bei denen ein Austrocknen der Haut ein wesentliches Krankheitsmerkmal darstellt (z.B. beim dyshidrotischen Ekzem).

Die erfindungsgemäßen Lipid-Hybridmoleküle können in der Kosmetik und in der Medizin zu therapeutischen Zwecken überall dort angewandt werden, wo der natürliche Aufbau biologischer Membranen durch pathologische Vorgänge gestört ist und sich durch den Einsatz dieser Lipid-Hybridverbindungen eine Stabilisierung der Membranstruktur und/oder eine Veränderung der Membraneigenschaften im Sinne eines therapeutischen Ziels (z. B. zur Erhöhung der Membranstabilität) erreicht werden soll.

Nachfolgend einige Beispiele für den therapeutischen Einsatz der Lipid-Hybridmoleküle:
- Nach derzeitiger Kenntnis sind Hauterkrankungen eines der Hauptgebiete für den Einsatz der genannten Lipid-Hybridmoleküle, insb. mit Ceramiden, gesättigten und ungesättigten Fettsäuren sowie Cholesterol und seinen Derivaten, nicht zuletzt deswegen, weil im Stratum corneum der menschlichen Haut die genannten Lipide eine wesentliche Rolle hinsichtlich Struktur und Funktion spielen.
- Im Fall bestimmter Vergiftungen, deren toxische Auswirkungen vorzugsweise die Leber betreffen, wie z.B. eine Vergiftung mit Tetrachlormethan (Tetrachlorkohlenstoff, TETRA, CCl₄), werden die Lipide und Lipid-analogen Verbindungen der Leberzellmembranen durch Radikale in ihrer Struktur angegriffen. Bei diesem Vorgang werden z.B. die Fettsäurereste der Lipide oxidiert, wodurch nach einer Reihe verschiedener Folgereaktionen die Kohlenstoffkette abgebaut wird. Die Folge hiervon ist ein partieller Abbau der Lipide und eine Destabilisierung der Membran, die zu einer teilweisen Auflösung der Zellmembran und damit einer schweren Schädigung der Zelle führt. Die Zufuhr der beschriebenen erfindungsgemäßen Verbindungen, z.B. von Fettsäure-enthaltenden Lipid-Hybridmolekülen, trägt in einem derartigen Vergiftungsfall zu einer deutlichen Stabilisierung der Membran der geschädigten Leberzellen bei.
- Eine Veränderung der Lipid-Zusammensetzung von Nervenzellen tritt bei einer großen Zahl unterschiedlicher pathologischer Schädigungen des Nervensystems auf. Hierzu gehören u. a. die Neuronopathie, die Axonopathie und die Myelinopathie. Als Ursachen für die Schädigung bzw. den Abbau der Lipid-reichen Myelinscheiden gilt u.a. die Einwirkung exogener Schadstoffe.
- Im Fall von Myelinopathien wie z.B. der Multiplen Sklerose kommen für eine Stabilisierung der Lipid-Membranen der Myelinscheiden wegen deren spezifischer Struktur vorzugsweise Lipid-Hybridmoleküle insb. mit Ceramiden, Freien Fettsäuren und Cholesterol in Betracht.
- In mehreren Untersuchungen konnte bisher nachgewiesen werden, dass die Anwesenheit von ω-3-mehrfach-ungesättigten Fettsäuren in Lipiden eine antithrombotische Wirkung besitzt. Hintergrund dieser Wirkung ist offensichtlich die bevorzugte Einlagerung dieser Lipid-Spezies in die Membranen von Blutzellen, insbesondere in Membranen der Blutplättchen, gegenüber derjenigen der Lipide mit ω-6-mehrfach-ungesättigten Fettsäuren. Die Anwendung von Fettsäure-haltigen Hybridmolekülen mit einem hohen Gehalt von ω-3-mehrfach-ungesättigten Fettsäuren bietet sich insb. in denjenigen Fällen an, in denen eine genetisch determinierte Fettstoffwechselstörung zu einem hohen thrombotischen, atherosklerotischen und cardiovaskulären Risiko führt.

## Patentansprüche

1. Lipid-Hybridmoleküle aus zwei Lipiden oder Lipid-analogen Verbindungen ausgewählt aus der Gruppe bestehend aus Ceramiden, Sphingosinen, Phospholipiden, Glykolipiden, Fettsäuren, Sterolen und deren Kombinationen, wobei die Lipide oder Lipid-analogen Verbindungen über ihr lipophiles Ende miteinander verknüpft sind und am hydrophilen Ende der Lipide oder Lipid-analogen Verbindungen mindestens eine hydrophile Gruppe zur Vergrößerung der Hydrathülle des Hybridmoleküls angeordnet ist.

2. Lipid-Hybridmolekül nach Anspruch 1,
**dadurch gekennzeichnet, dass** die hydrophile Gruppe ausgewählt ist aus der Gruppe bestehend aus
Aminosäuren, insbesondere Aminosäuren mit polaren Gruppen oder Ladungen, bevorzugt ausgewählt aus der Gruppe bestehend aus -COOH, -NH₂, -COO⁻ und -NH₃⁺, bevorzugt Serin, Threonin, Lysin, Arginin, Histidin, Asparaginsäure, Glutaminsäure, Asparagin, Glutamin, Tyrosin und Tryptophan.
Polyole, insbesondere Ethandiol oder Glycerin, Zucker, insbesondere Glucose oder Galaktose, Zuckerderivate mit einer dissoziierbaren
-COOH-Gruppe, insbesondere Glucuronsäure oder Galakturonsäure, oder Aminozucker,
organische Säuren, insbesondere Di- bzw. Tricarbonsäuren, bevorzugt Malonsäure, Bernsteinsäure, Äpfelsäure oder Citronensäure
anorganischen Säuren, insbesondere Schwefel- oder Phosphorsäuren,
Cholin,
Harnstoff oder Harnstoffderivate
sowie Kombinationen hiervon.

3. Lipid-Hybridmoleküle nach einem der vorhergehenden Ansprüche,
**dadurch gekennzeichnet, dass** die Bindung der hydrophilen Gruppe im Fall der Ceramide und der Glykolipide (Cerebroside) entweder über die Hydroxylgruppe oder die Hydroxymethylengruppe des Sphingosingrundkörpers, im Fall der Fettsäuren über die OH-Gruppe der Carboxylgruppierung, im Fall der Sterole über die 3-ständige OH-Gruppe und im Fall der Phospholipide über die in 1-Position des Glycerins gebundene Phosphatgruppe erfolgt.

4. Lipid-Hybridmolekül nach dem vorhergehenden Anspruch,
**dadurch gekennzeichnet, dass** das Hybridmolekül insbesondere folgende Lipide oder Lipid-analoge Verbindungen umfassen:
Ceramid-Sphingosin, Ceramid-Phospholipid, Ceramid-Glykolipid, Ceramid-Fettsäure, Ceramid-Sterol, Sphingosin-Sphingosin, Sphingosin-Phospholipid, Sphingosin-Glykolipid, Sphingosin-Fettsäure, Sphingosin-Sterol, Phospholipid-Phospholipid, Phospholipid-Glykolipid, Phospholipid-Fettsäure, Phospholipid-Sterol, Glykolipid-Glykolipid, Glykolipid-Fettsäure und Glykolipid-Sterol.

5. Lipid-Hybridmolekül nach einem der vorhergehenden Ansprüche,
**dadurch gekennzeichnet, dass** die Ceramid-Moleküle ausgewählt sind aus der Gruppe bestehend aus Ceramid-1, Ceramid-2, Ceramid-3, Ceramid-4, Ceramid-5, Ceramid-6, Ceramid-7, Ceramid-8, Ceramid-9 sowie deren ω-Hydroxyceramiden.

6. Lipid-Hybridmolekül nach einem der vorhergehenden Ansprüche,
**dadurch gekennzeichnet, dass** die Lipide oder Lipid-analogen Verbindungen über ihr lipophiles Ende kovalent miteinander verbunden sind.

7. Lipid-Hybridmolekül nach einem der vorhergehenden Ansprüche,
**dadurch gekennzeichnet, dass** die kovalente Bindung der Lipide oder Lipid-analogen Verbindungen über ihr lipophiles Ende im Fall der Ceramide und der Glykolipide (Cerebroside) entweder über die Alkylkette der Sphingosin-Grundstruktur oder über den Fettsäurerest oder über die Kombination beider Angriffspunkte, im Fall der Fettsäuren über die an der Alkylkette endständige Methylgruppe, im Fall der Sterole und ihrer Derivate (Ester) über eine der beiden endständigen Methylgruppen der in Position 17 der Sterolstruktur stehenden verzweigten Alkylkette und im Fall der Phospholipide über die jeweils endständige Methylgruppe einer der beiden in der 2-Position oder 3-Position des Glycerins stehenden Fettsäurereste oder über die Kombination beider Angriffspunkte erfolgt.

8. Lipid-Hybridmolekül nach einem der vorhergehenden Ansprüche,
**dadurch gekennzeichnet, dass** die Lipide oder Lipid-analogen Verbindungen über einen Spacer verbunden sind.

9. Lipid₋Hybridmolekül nach dem vorhergehenden Ansprüche,
**dadurch gekennzeichnet, dass** der Spacer aus mindestens einem Atom ausgewählt aus der Gruppe bestehend aus Kohlenstoff, Stickstoff oder Sauerstoff oder Kombinationen hiervon besteht oder diese enthält.

10. Lipid-Hybridmolekül nach dem vorhergehenden Ansprüche,
**dadurch gekennzeichnet, dass** der Spacer vorzugsweise aus einem Sauerstoffatom oder einer -O-(CH₂)ₙ-O-Gruppe mit n = 1 bis 20 besteht.

11. Lipid-Hybridmolekül nach einem der vorhergehenden Ansprüche,
**dadurch gekennzeichnet, dass** die Lipide oder Lipid-analogen Verbindungen ausgewählt sind aus der Gruppe bestehend aus Ceramiden, Sphingosinen, Phospholipiden, Glykolipiden, Fettsäuren, Sterolen und deren Kombinationen.

12. Lipid-Hybridmolekül nach einem der vorhergehenden Ansprüche zur Verwendung als Arzneimittel.

13. Lipid-Hybridmolekül nach einem der Ansprüche 1 bis 10 zur Herstellung eines Arzneimittels zur Behandlung von Erkrankungen, bei denen eine Störung der Lipidzusammensetzung der Zellmembranen eines Organismus hinsichtlich ihres Gehalts an Lipiden oder Lipid-analogen Verbindungen vorliegt.

14. Lipid-Hybridmolekül nach einem der Ansprüche 1 bis 11 zur Herstellung eines Arzneimittels zur Behandlung von Erkrankungen, bei denen eine Störung der Zusammensetzung der Lipid-Doppelschichten des Stratum corneum der Haut hinsichtlich ihres Gehaltes an Lipiden oder Lipid-analogen Verbindungen vorliegt.

15. Verwendung des Lipid-Hybridmoleküls nach einem der Ansprüche 1 bis 11 zur Herstellung einer kosmetischen Zubereitung, insbesondere als Creme, Salbe, Lotion, Emulsion, Gel, Spray, kosmetisches Öl oder Liposomen.
